# EUROPEAN PATENT APPLICATION

(11) **EP 3 175 811 A1**
(43) Date of publication of application: **07.06.2017**
(21) Application number: 15828083.4
(22) Date of filing: 14.05.2015
(51) Int. Cl.: A61B 34/00, B25J 3/00

(54) **MEDICAL MANIPULATOR AND TREATMENT INSTRUMENT PACKAGE**

(30) Priority: 31.07.2014 JP 2014156152
(71) Applicant: Olympus Corporation, Hachioji-shi Tokyo 192-8507 (JP)
(72) Inventor: IIDA, Masatoshi, Tokyo 192-8507 (JP); KOMURO, Takahiro, Tokyo 192-8507 (JP)
(74) Representative: Gunzelmann, Rainer
(86) International application number: PCT/JP2015/063932
(87) International publication number: WO 2016/017237

(57) **Abstract**

A medical manipulator (1) has master units (11A, 11B) and treatment tools (2A, 2B). The master units (11A, 11B) each have a storage unit (115). The storage unit (115) stores correspondence relationship between each input mechanism (111, 112) of the master units (11A, 11B) and each part (211, 212, 221, 222) of the treatment tools (2A, 2B). When the master units (11A, 11B) and the treatment tools (2A, 2B) are connected to a controller (15), the controller (15) saves information in the storage unit (115) on a correspondence information saving unit (152). When the information associates a button (112) with a knife unit (221), the controller (15) supplies high-frequency current to the knife unit (221) as an operator (Op) presses the button (112). Because the storage unit (115) is provided at the master units (11A, 11B) rather than the treatment tools (2A, 2B), even when blood, a body fluid, or the like adheres to the treatment tools (2A, 2B), it is possible to reliably provide information in the storage unit (115) to the controller (15).

## Description

### Technical Field

The present invention relates to a medical manipulator and a treatment tool package.

Priority is claimed on Japanese Patent Application No. 2014-156152, filed on July 31, 2014, the content of which is incorporated herein by reference.

### Background Art

Conventionally, as a medical manipulator, a medical manipulator equipped with a master by which an operator performs a manipulation input, and a slave arm that operates in accordance with the manipulation input to the master is known.

Usually, in such a medical manipulator, in order to enable various procedures, manipulations, and the like, a plurality of treatment tools equipped with different end effectors are prepared, and one of the treatment tools is appropriately selected and mounted on a slave arm and the treatment tools are exchanged to perform various procedures and the like on a patient.

To suitably operate a plurality of types of treatment tools with different end effectors by a single slave arm, there is a need to provide operating parameters corresponding to each of the treatment tools to a mechanism for driving the slave arm, and to perform driving in accordance with the operating parameters. Patent Literature 1 discloses a configuration in which a memory that stores information for recognizing a type of a treatment tool, operating parameters, and the like is mounted. When the treatment tools are mounted, information stored in the memory is sent to a control unit that controls the driving of the slave arm, and the control unit is capable of recognizing the type of the treatment tool and suitably driving the treatment tool using the sent operating parameters.

As another example, Patent Literature 2 discloses a configuration in which a barcode including information for recognizing the type is attached to the treatment tool. A camera is provided at a mounting portion of the driving side mechanism on which the treatment tool is mounted. When the treatment tool is mounted on the driving side mechanism, the barcode is disposed at a position where the barcode can be photographed by the camera. When the barcode acquired by the camera is read, the control unit recognizes the type of the treatment tool.

### Citation List

### Patent Literature

Patent Literature 1: United States Patent No. 7,048,745
Patent Literature 2: Japanese Unexamined Patent Application, First Publication No. 2009-226028

### Summary of Invention

### Technical Problem

In both of Patent Literatures 1 and 2, a configuration including the information necessary for the operation is provided in the treatment tool. Because the treatment tool is used such that a distal end portion provided with an end effector or the like is inserted into the patient's body, there is a possibility of blood, a body fluid, or the like flowing backward and damaging the memory or staining the barcode. There is risk that such damage to the memory or staining of the barcode may cause malfunction of the medical manipulator, especially when the treatment tool is sterilized, etc. after use and re-used.

Also, in the medical manipulator, usually, a slave arm in an unclean (non-sterile) state is covered with a drape, and the treatment tool and the slave arm are connected to each other through the drape (or a sterilized adapter). In this case, since the memory needs to be electrically connected to the slave via the drape, a rise in the cost due to including an electrode in the drape, and degradation of reliability of communication caused by defective connection of the electrode of the drape may occur.

In the case of the barcode, because the barcode scanner in the unclean state is brought close to the treatment tool upon reading, the treatment tool may come into contact with the barcode scanner and become unclean.

In view of the above circumstances, an object of the present invention is to provide a medical manipulator that is capable of reliably providing the operating parameters and the like corresponding to the treatment tool to the control unit, without being affected by the backflow of blood, body fluids, or the like.

Another object of the present invention is to provide a treatment tool package that is suitably applicable to the medical manipulator.

### Solution to Problem

According to a first aspect of the present invention, a medical manipulator, which is equipped with a manipulation input unit by which an operator performs a manipulation input, and a plurality of treatment tools that are driven in accordance with the manipulation input, includes: a storage unit having correspondence information used when a driving signal that drives the treatment tool is generated in accordance with the manipulation input. The storage unit is disposed at the manipulation input unit.

According to a second aspect of the present invention, in the medical manipulator according to the first aspect, the storage unit may be attachable to and detachable from the manipulation input unit.

According to a third aspect of the present invention, in the medical manipulator according to the first aspect, the storage unit may be a barcode that is printed on the manipulation input unit.

According to a fourth aspect of the present invention, in the medical manipulator according to any one of the first to third aspects, the treatment tool and the manipulation input unit may be disposable elements that are discarded after use.

According to a fifth aspect of the present invention, in the medical manipulator according to any one of the first to fourth aspects, the storage unit may have a plurality of pieces of the correspondence information.

According to a sixth aspect of the present invention, a medical manipulator, which is equipped with a manipulation input unit by which an operator performs a manipulation input, and a treatment tool that is driven in accordance with the manipulation input, includes: a controller to which the treatment tool is connected; and a storage unit having correspondence information used when a driving signal that drives the treatment tool is generated in accordance with the manipulation input. The storage unit is attachable to and detachable from the controller.

According to a seventh aspect of the present invention, a medical manipulator, which is equipped with a manipulation input unit by which an operator performs a manipulation input, and a treatment tool that is driven in accordance with the manipulation input, includes: a driving unit to which the treatment tool is connected; and a storage unit having correspondence information used when a driving signal that drives the treatment tool is generated in accordance with the manipulation input. The storage unit is attachable to and detachable from the driving unit.

According to an eighth aspect of the present invention, a treatment tool packages used in a medical manipulator, includes: a manipulation input unit by which an operator performs a manipulation input; a treatment tool that is driven in accordance with the manipulation input; a storage unit having correspondence information used when a driving signal that drives the treatment tool is generated in accordance with the manipulation input; and a packaging material that houses the treatment tool and the manipulation input unit.

According to a ninth aspect of the present invention, in the treatment tool package according to the eighth aspect, the storage unit may be provided at the manipulation input unit.

According to a tenth aspect of the present invention, in the treatment tool package according to the eighth aspect, the storage unit may be a storage medium that is housed in the packaging material.

According to an eleventh aspect of the present invention, in the treatment tool package according to the eighth aspect, the storage unit may be a barcode that is printed on the packaging material.

### Advantageous Effects of Invention

According to the medical manipulator of the present invention, it is possible to reliably provide the operating parameters and the like corresponding to the treatment tool to the control unit, without being affected by the backflow of blood, body fluids, or the like.

Further, the treatment tool package of the present invention is suitably applicable to the medical manipulator.

### Brief Description of Drawings

Fig. 1 is a diagram showing an example of a medical manipulator system to which a medical manipulator according to the present invention is applied.
Fig. 2 is a diagram showing a partial configuration of a medical manipulator according to a first embodiment of the present invention.
Fig. 3 is a diagram showing a treatment tool package in the medical manipulator.
Fig. 4 is a functional block diagram of a controller in the medical manipulator.
Fig. 5 is a diagram showing a modified example of the treatment tool package.
Fig. 6 is a diagram showing a treatment tool package in a medical manipulator according to a second embodiment of the present invention.
Fig. 7 is a diagram showing a treatment tool package in a medical manipulator according to a third embodiment of the present invention.
Fig. 8 is a diagram showing a correspondence relationship between a master unit and a treatment tool in first correspondence information.
Fig. 9 is a diagram showing a correspondence relationship between the master unit and the treatment tool in second correspondence information.

### Description of Embodiments

### (First embodiment)

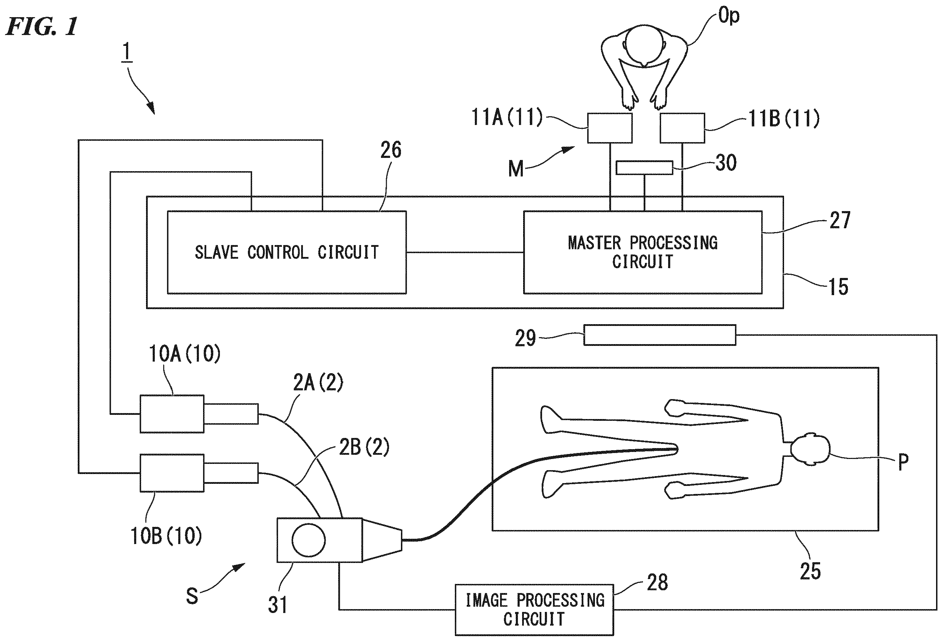

A first embodiment of the present invention will be described below with reference to Figs. 1 to 5, but an example of a medical manipulator system to which a medical manipulator according to the present embodiment is applied will be described before that.

Fig. 1 shows an example of a master slave type medical manipulator system to which a medical manipulator 1 according to the present embodiment is applied. The master slave type medical manipulator system is a system that includes a master M serving as an input unit for receiving a manipulation input from a physician, and a slave S serving as a treatment portion for performing a treatment on a patient, and remotely controls the slave S to follow the manipulation input of the master M.

The medical manipulator system shown in Fig. 1 has a surgical table 25 on which a patient P is placed, an endoscope 31 and a treatment tool 2 to be inserted into the patient P, a slave control circuit 26, a manipulation input unit 11 that is manipulated by an operator Op, a master processing circuit 27, an image processing circuit 28, and a display 29. In the present embodiment, the endoscope 31 and the treatment tool 2 correspond to the slave S, and manipulation input units 11A and 11B and a setting manipulation unit 30 correspond to the master M.

The surgical table 25 is a table on which the patient P to be observed or treated is placed. The endoscope 31 and the treatment tool 2 are installed near the surgical table 25. Further, the endoscope 31 and the treatment tool 2 may be installed on the surgical table 25.

The treatment tool 2 is configured to have a plurality of joints with multiple degrees of freedom. The treatment tool 2 performs treatment on the patient P placed on the surgical table 25 by bending the respective joints or by driving an end effector provided at the distal end portion. The respective joints are individually driven by the driving unit 10. As the driving unit 10, it is possible to use, for example, motor units (driving units) 10A and 10B that have a servo mechanism equipped with an incremental encoder, a decelerator, or the like. Operation control of the driving unit 10 is performed by the slave control circuit 26.

An insertion section of the treatment tool 2 may be a rigid section having no flexibility, or may be a flexible section having flexibility. Further, as the treatment tool 2, it is possible to suitably select and adopt a treatment tool that operates the joint, the end effector, and the like by pushing and pulling a rigid rod, and a treatment tool that operates the joint, the end effector, and the like by a wire or the like having flexibility. Even when the treatment tool 2 is equipped with a rigid insertion section, the joint, the end effector, and the like may be driven by a wire or the like. In the example shown in Fig. 1, the treatment tool 2 is equipped with a flexible insertion section, and has a configuration in which the joint, the end effector, and the like is driven by a wire or the like.

The treatment tool 2 is inserted through a treatment tool channel (not shown) of the endoscope 31, and is inserted into the body of the patient P. The endoscope 31 and the treatment tool 2 inserted through the endoscope 31 are introduced into the body, for example, from a natural opening of the patient P such as the mouth or anus and through the alimentary canal or the like. The treatment tool 2 may be inserted through an overtube that is attached to the endoscope 31.

The slave control circuit 26 is configured to have, for example, a CPU, a memory, and the like. The slave control circuit 26 stores a predetermined program for controlling the treatment tool 2. The slave control circuit 26 controls the operation of the treatment tool 2 in accordance with control signals from the master processing circuit 27. That is, the slave control circuit 26 specifies the treatment tool 2 of the manipulation target of the master M manipulated by the operator Op, based on the control signals from the master processing circuit 27. The slave control circuit 26 calculates a driving amount that is necessary for moving the specified treatment tool 2 in accordance with the master manipulation amount of the operator Op.

Further, the slave control circuit 26 controls the operation of the treatment tool 2 associated with the manipulation input unit 11 in accordance with the calculated driving amount. At this time, the slave control circuit 26 inputs the driving signal to the driving unit 10 connected to the associated treatment tool, and controls the magnitude and the polarity of the driving signal so that the driving amount of the treatment tool 2 of the manipulation target becomes a target driving amount, in accordance with the detection signal that is input from a position detector of a power unit depending on the operation of the associated driving unit 10.

The medical manipulator 1 includes, as the manipulation input unit 11, master units 11A and 11B each having an input mechanism such as a plurality of switches or a joystick. The input to the master unit such as pressing of the switches, inclining of the joystick, or the like by the operator Op is detected by the master processing circuit 27 as the manipulation input to the manipulation input unit 11. The manipulation input unit 11 is not limited to a configuration that includes the master unit, and may be configured to have, for example, a link mechanism. In this case, for example, a position detector such as an incremental encoder is provided for each link that constitutes the link mechanism. By the position detector detecting the operation of each link, the manipulation input to the manipulation input unit 11 is detected in the master processing circuit 27.

The setting manipulation unit 30 has various manipulation members, such as a clutch button for switching the control connection/disconnection between the master M and the slave S, a scaling change switch for changing an operation ratio of the master M and the slave S, and a foot switch for stopping the system in an emergency. When one of the manipulation members constituting the setting manipulation unit 30 is manipulated by the operator Op, the manipulation signal corresponding to the manipulation of the corresponding manipulation member is input to the master processing circuit 27 from the setting manipulation unit 30.

The master processing circuit 27 analyzes the manipulation signal from the manipulation input unit 11 and the manipulation signal from the setting manipulation unit 30. The master processing circuit 27 generates a control signal for controlling the medical manipulator system in accordance with the analysis result of the manipulation signal and inputs the control signal to the slave control circuit 26.

The image processing circuit 28 performs various image processes for displaying the image signal that is input from the endoscope 31, and generates the displaying image data on a display 29. The display 29, for example, is made up of a liquid crystal display, and displays an image based on the image data generated by the image processing circuit 28 in accordance with the image signals obtained via the endoscope 31.

In the medical manipulator system configured as described above, when the operator Op manipulates the manipulation input unit 11, the corresponding treatment tool 2 operates in response to the movement of the manipulation input unit 11. Thus, it is possible to perform a desired procedure on the patient P.

In the medical manipulator 1, a plurality of types of treatment tools with the different end effectors and joint degrees of freedom are prepared. The operator Op uses the treatment tool appropriately selected in consideration of the part to be treated, the content of the treatment, and the like, with the selected treatment tool mounted on the driving unit.

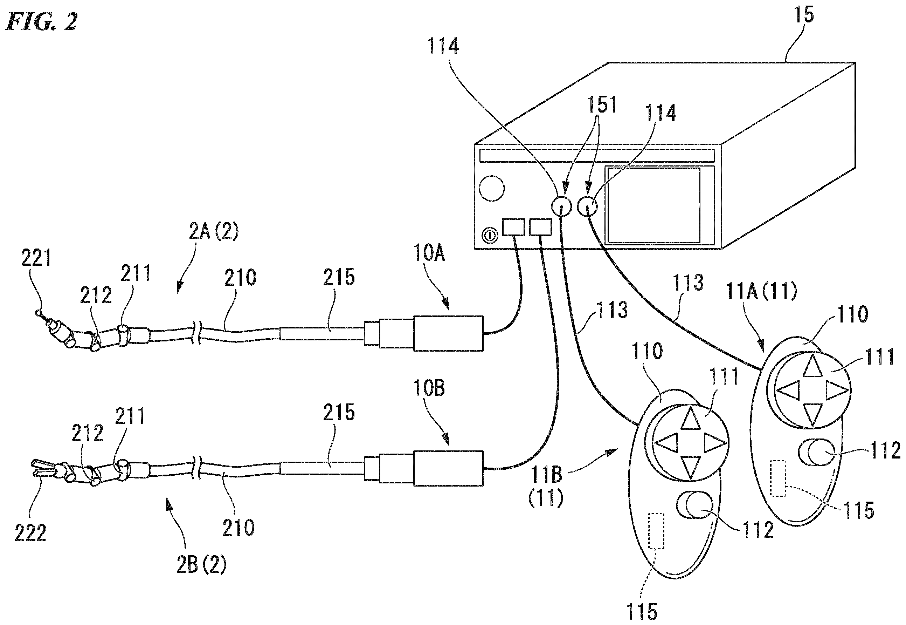

Fig. 2 shows a partial configuration of the medical manipulator 1 according to the present embodiment. Fig. 2 shows a controller 15 that stores the slave control circuit 26 and the master processing circuit 27, the treatment tools 2A and 2B connected to the controller 15, and the master units 11A and 11B.

The treatment tools 2A and 2B have a common basic structure, and each include a flexible insertion section 210, joints 211 and 212 connected to the distal end side of the insertion section 210, an end effector connected to the joint 212, and a connection section 215 provided at a proximal end side of the insertion section 210. Wires are attached to the respective joints 211 and 212. Each wire extends to the interior of the connection section 215 through the interior of the insertion section 210.

The treatment tools 2A and 2B have the different end effectors. The end effector of the treatment tool 2A is an energizable knife unit 221. The end effector of the treatment tool 2B is an openable and closable grasping forceps 222. A power supplying wire for supplying a high frequency current to the knife unit 221, and a wire for driving the grasping forceps 222 extend to the interior of the connection section 215 through the insertion section 210.

The connection section 215 is connected to one of the motor units 10A and 10B that are connected to the controller 15. Each motor unit has a plurality of motors. When the connection section 215 and the motor unit are connected to each other, the wires are connected to the respective different motors and the driving force generated by the motor unit can be transmitted to each wire inside the connection section 215. Further, it is also possible to supply the high-frequency current to the knife unit 221 from the controller 15.

The master units 11A and 11B have a common basic structure, and each includes a main body 110 grasped by the operator Op, a direction switch 111 and a button 112 that function as an input mechanism provided in the main body 110, and a code 113 that extends from the main body 110. The direction switch 111 is capable of performing the manipulation inputs of a total of four directions of up and down parallel to the longitudinal direction of the main body 110, and left and right parallel to the width direction of the main body 110. The master units 11A and 11B can be connected to the controller 15 by connecting a terminal 114 provided at an end portion of the cord 113 to a jack 151 provided in the controller 15. When the master unit and the controller are connected to each other, the manipulation input to the input mechanism of the master unit can be sent to the controller.

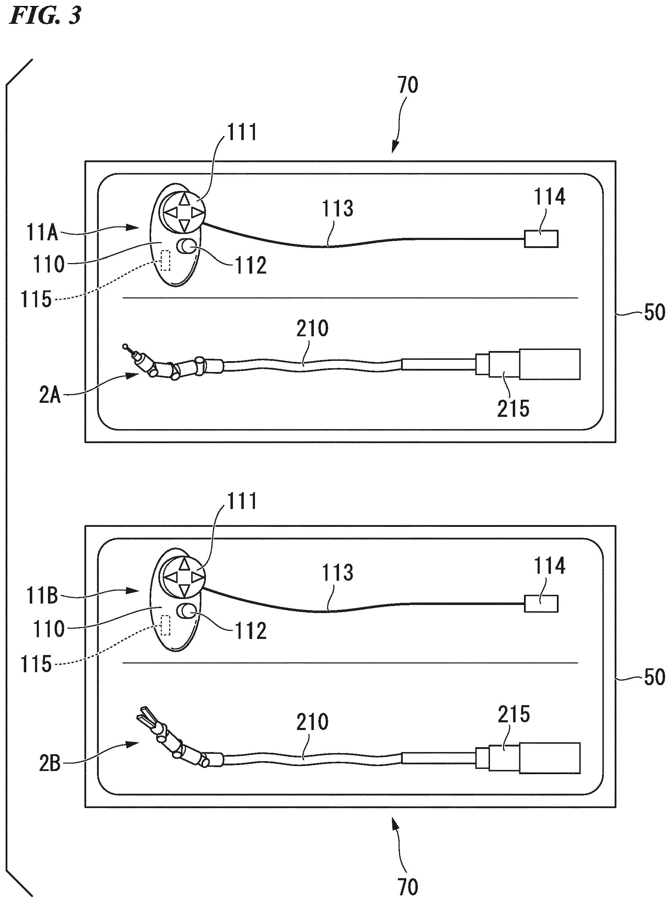

In the present embodiment, the treatment tool and the master unit are disposable units that are discarded after use. As shown in Fig. 3, the treatment tool and the master unit are sold and supplied in a treatment tool package 70 in which the treatment tool (e.g., the treatment tool 2A) and the corresponding master unit (e.g., the master unit 11A) are housed in the same packaging material 50.

The treatment tool 2A and the treatment tool 2B are manipulated by the master unit of the same configuration. The treatment tools are alike in that the joints 211 and 212 are driven by the direction switch 111, but the driving content of the controller 15 for the input to the button 112 are different from each other. That is, in the treatment tool 2A, the supply of the high-frequency current to the knife unit 221 is turned on and off by the input to the button 112. In the treatment tool 2B, the grasping forceps 222 is opened and closed by the input to the button 112.

Modes in which the treatment tool is driven with respect to the manipulation input to each input mechanism of the master unit are different for each treatment tool. Therefore, when the correspondence information about the correspondence between the manipulation input and the driving mode is not appropriately applied, the controller 15 may not appropriately operate the connected treatment tool. In the present embodiment, the correspondence information is stored in the master unit that is packaged with the treatment tool. Specifically, the correspondence information is stored on a storage unit 115 (see Fig. 2) that is provided in the main body 110. The correspondence information may include parameters about the quantitative relation (scaling) between the manipulation amount of the input mechanism and the driving amount of the corresponding part, in addition to the parameters about the correspondence relationship between the respective input mechanisms and the respective portions of the treatment tool.

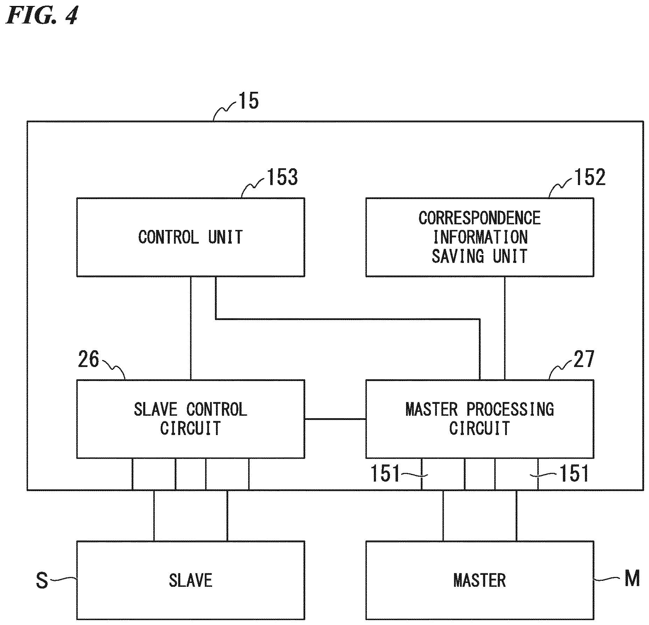

Fig. 4 shows a functional block diagram of the controller 15. The controller 15 includes a correspondence information saving unit 152 for saving the correspondence information, and a control unit 153 for controlling the whole controller 15, in addition to the slave control circuit 26 and the master processing circuit 27 described above. As the correspondence information saving unit 152, it is possible to use various rewritable storage media or the like. A CPU, a program, and the like can be used as the control unit 153.

A preparatory operation prior to use in the medical manipulator 1 having the aforementioned configuration will be described.

First, the operator starts the controller 15, and connects the treatment tool and the master unit included in the treatment tool package 70 to the controller 15. At this time, the controller 15 cannot recognize the number and positional relationships of the joints provided in the treatment tool, the type of the end effectors, and the like. Meanwhile, the correspondence information is sent to the controller 15 from the storage unit 115 of the master unit, and is saved on the correspondence information saving unit 152.

The control unit 153 sets the correspondence between the direction switch 111 and the button 112 serving as the input mechanisms of the master unit and each part of the treatment tool 2A on the basis of the correspondence information saved on the correspondence information saving unit 152. For example, the input in the up and down directions of the direction switch 111 and the driving of the joint 212 are set to be associated with each other, the input in the left and right directions of the direction switch 111 and the driving of the joint 211 are set to be associated with each other, and the manipulation of the button 112 and turning on and off the supply of the high-frequency current to the knife unit 221 are set to be associated with each other.

When the control unit 153 sends the setting contents to the slave control circuit 26 and the master processing circuit 27, the preparatory operation prior to use of the medical manipulator 1 is completed. The completion of the preparatory operation prior to use may be reported to the operator Op by the display of the display 29, the sound, or the like.

After the preparatory operation is completed, when the operator Op performs the manipulation input to the master unit, the control unit 153 controls the generation of the driving signals based on the correspondence information , and the treatment tool is suitably driven.

When a plurality of treatment tools and a plurality of master units are connected to the controller 15, a configuration that enables a change in the correspondence relationship between the treatment tools and the master units may be provided as required. In this case, for example, a configuration in which a list of the motor unit to which the treatment tool is connected, and a list of the connected master unit are displayed on the display 29, and the control unit 153 performs the setting, while using the correspondence information saved on the correspondence information saving unit 152 in accordance with the correspondence relationship specified by the operator may be provided.

As described above, according to the medical manipulator 1 according to the present embodiment, the correspondence information necessary for suitably driving each part of the treatment tool 2 is stored in the master units 11A and 11B as the manipulation input unit 11, rather than the treatment tool. Therefore, even when blood, a body fluid, or the like flows backward to the insertion section during use of the treatment tool, the acquisition of the correspondence information in the controller 15 and the setting operation of the control unit 153 based on the correspondence information are not affected. As a result, it is possible to suitably provide the operating parameters or the like corresponding to the treatment tool to the driving side, without being affected by blood, a body fluid, or the like.

Further, since all the exchanges of correspondence information are performed on the unclean side, it is possible to exchange the information without passing through the drape. As a result, it is possible to reliably perform the exchange of the correspondence information.

Further, in the treatment tool package 70 according to the present embodiment, the treatment tool, the master unit capable of manipulating the treatment tool, and the storage unit having the correspondence information are contained in a single packaging material 50. That is, in the treatment tool package 70, a physical correspondence relationship between the treatment tool and the manipulation input unit is secured. Accordingly, the user can suitably establish the correspondence relationship between the treatment tool and the manipulation input unit, by merely connecting the treatment tool and the master unit, which are included in the same package, to the controller, and it is possible to facilitate the handling.

In the present embodiment, an example in which a single treatment tool and a single master unit are packaged as a set in the treatment tool package has been described. However, when it is possible to manipulate a plurality of treatment tools by a single master unit, the treatment tool may be sold and supplied in a different manner.

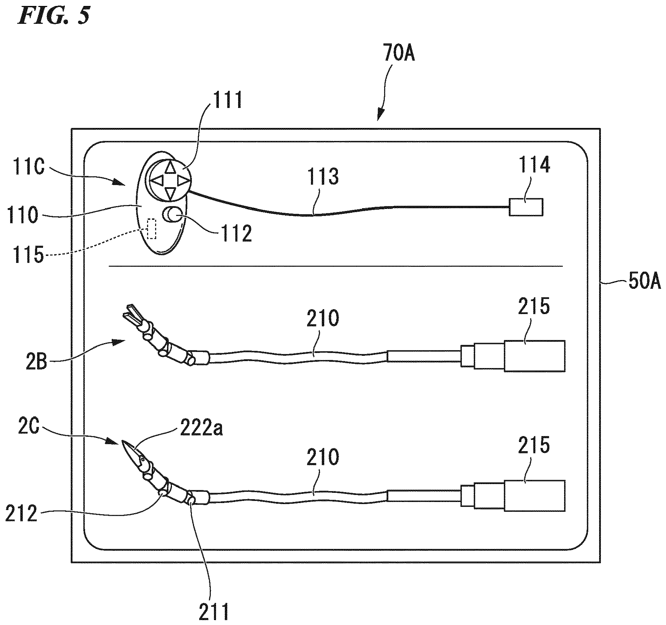

In a treatment tool package 70A of a modified example shown in Fig. 5, a single master unit 11C and two treatment tools 2B and 2C are housed in a single packaging material 50A in the treatment tool package. The end effector of the treatment tool 2C is a grasping forceps 222a, and the treatment tool 2C is the same as the treatment tool 2B in that the end effector is a grasping forceps. However, a pair of opening and closing jaws of the grasping forceps 222a is smaller than a pair of jaws of the grasping forceps 222, and the grasping forceps 222a is configured under the assumption that it will perform a more detailed manipulation. Further, the treatment tool 2C has joints 211 and 212 similar to the treatment tool 2B, but the scaling in the correspondence information is also different from the treatment tool 2B so that it can suitably perform the more detailed manipulation.

On the storage unit 115 of the master unit 11C that is housed in the packaging material 50A, two kinds of correspondence information including the correspondence information about the treatment tool 2B and the correspondence information about the treatment tool 2C are stored. When the master unit 11C is connected to the controller 15, both of the two types of the correspondence information is saved on the correspondence information saving unit 152. Since the control unit 153 cannot determine which types of the correspondence information should be used to perform the setting with respect to the treatment tool connected to the controller 15, the control unit 153 displays the list of the treatment tools on the display 29, and asks the operator Op to select the suitable treatment tool. When the operator selects the treatment tool from an interface or the like (not shown) provided in the master unit or the controller 15, the control unit 153 performs the setting in accordance with the correspondence information of the selected treatment tool.

In a configuration such as the aforementioned modified example, since it is possible to share the master unit in the treatment tools with the same basic manipulation, it is possible to reduce the manufacturing cost of the treatment tool package. Further, it is possible to reduce the frequency of exchanging the master unit with the exchange of the treatment tool, thereby improving the manipulation property.

As another modified example, a plurality of master units and a plurality of treatment tools may be housed in a single treatment tool package. In this case, a plurality of sets of a master unit and a treatment tool of one-to-one correspondence may be housed, and a plurality of master units such as the master unit 11C in which the correspondence information about the entire plurality of treatment tools is stored may be housed.

### (Second embodiment)

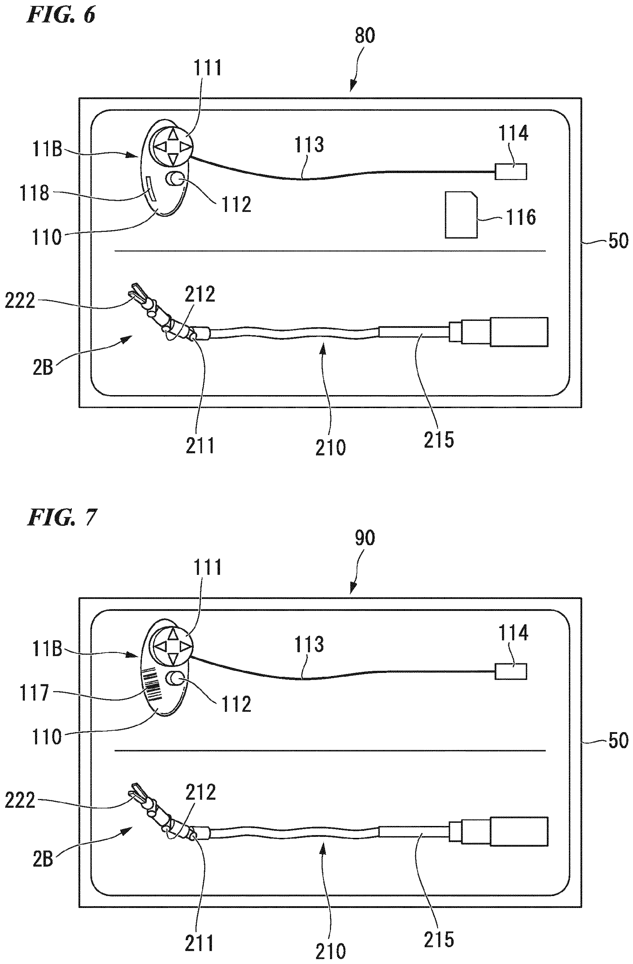

Next, a second embodiment of the present invention will be described with reference to Fig. 6. The present embodiment differs from the first embodiment in that the storage unit is attachable to and detachable from the master unit. Further, in the following description, the configurations that are common to those already described are denoted by the same reference numerals, and the repeated description will not be provided.

Fig. 6 is a diagram showing a treatment tool package 80 in a medical manipulator according to the present embodiment. The treatment tool package 80 is the same as in the first embodiment in that a set of the master unit and the treatment tool is housed in the packaging material 50. However, an SD memory card 116 that is a portable storage medium is used as the storage unit. The SD memory card 116 is housed in the packaging material 50 separately from the master unit and the treatment tool. The SD memory card 116 can be mounted on the master unit by being inserted into a slot 118 provided in the main body 110 of the master unit.

The preparatory operation prior to use of the medical manipulator according to the present embodiment is the same as the first embodiment, except that a master unit in which the SD memory card 116 is inserted into the slot 118 is connected to the controller 15. The SD memory card 116 may be inserted into the slot 118 after connecting the master unit to the controller 15.

In the medical manipulator according to the present embodiment using the treatment tool package 80, it is also possible to suitably provide the operating parameters or the like corresponding to the treatment tool to the driving side, without being affected by blood, a body fluid, or the like, as in the first embodiment.

Further, since the SD memory card 116 that is a portable storage medium capable of being attached to and detached from the master unit is used as the storage unit, it is possible to produce a master unit in a highly versatile state. As a result, it is possible to reduce the manufacturing cost, and it is possible to easily cope with updating or the like of the correspondence information.

In the present embodiment, the portable storage medium used in the storage unit can be appropriately selected from various known portable storage media, without being limited to the SD memory card. Further, the location at which the storage unit is mounted is not limited to the master unit, and the storage unit may be mounted at a location other than the treatment tool. For example, the storage unit may be mounted by providing a slot in the controller, the motor unit, or the like. In such a case, it is also possible to provide a configuration in which only the storage unit and the treatment tool are housed in the treatment tool package and the manipulation input unit is not disposable.

Furthermore, as in the aforementioned modified example, a plurality of types of correspondence information may be stored on the storage unit. In this case, the plurality of types of correspondence information may be stored on a single storage medium, and the plurality of storage media on which a piece of correspondence information is stored may be mounted.

### (Third embodiment)

Next, a third embodiment of the present invention will be described with reference to Fig. 7. The present embodiment differs from each of the aforementioned embodiments in an aspect of the storage unit.

Fig. 7 is a diagram showing a treatment tool package 90 in a medical manipulator according to the present embodiment. The treatment tool package 90 is the same as the first embodiment in that a set of the master unit and the treatment tool is housed in the packaging material 50. However, a barcode 117 which functions as a storage unit is printed on the surface of the main body 110 of the master unit. A controller (not shown) is provided with a reading unit such as a scanner capable of reading the barcode 117.

The preparatory operation prior to use of the medical manipulator according to the present embodiment is the same as the first embodiment, except that the correspondence information is saved on the correspondence information saving unit 152 by reading the barcode 117 using the reading unit provided in the controller.

In the medical manipulator according to the present embodiment using the treatment tool package 90, as in each of the aforementioned embodiments, it is also possible to suitably provide the operating parameters or the like corresponding to the treatment tool to the driving side, without being affected by blood, a body fluid, or the like.

Further, because the barcode 117 printed on the master unit is used as the storage unit, it is possible to reduce the manufacturing cost by simplifying the structure of the master unit, and it is possible to easily cope with the updating or the like of the correspondence information.

In the present embodiment, the barcode used as the storage unit is not limited to a one-dimensional code, and a two-dimensional code may be used. Because the two-dimensional code can have more information than the one-dimensional code, the two-dimensional code is effective for a case in which the operating parameters included in the correspondence information are large or a case in which a plurality of types of correspondence information are to be included. When the plurality of types of correspondence information are stored on the storage unit, a plurality of one-dimensional codes may be used.

Further, the location at which the barcode is printed is not limited to the master unit, and the barcode may be mounted at a location other than the treatment tool. For example, the barcode may be printed on the packaging material of the treatment tool package. In such a case, it is possible to provide a configuration in which only the treatment tool is housed in the packaging material on which the barcode is printed, and the manipulation input unit is not disposable.

While embodiments of the present invention have been described, the technical scope of the present invention is not limited to the aforementioned embodiments, and it is possible to change the combination of components or to variously change or omit each component without departing from the scope of the present invention.

For example, in the above description, while an example in which there is a piece of correspondence information for each treatment tool has been described, a plurality of types of correspondence information may be set for each treatment tool and may be stored on the storage unit to meet various demands of the operator.

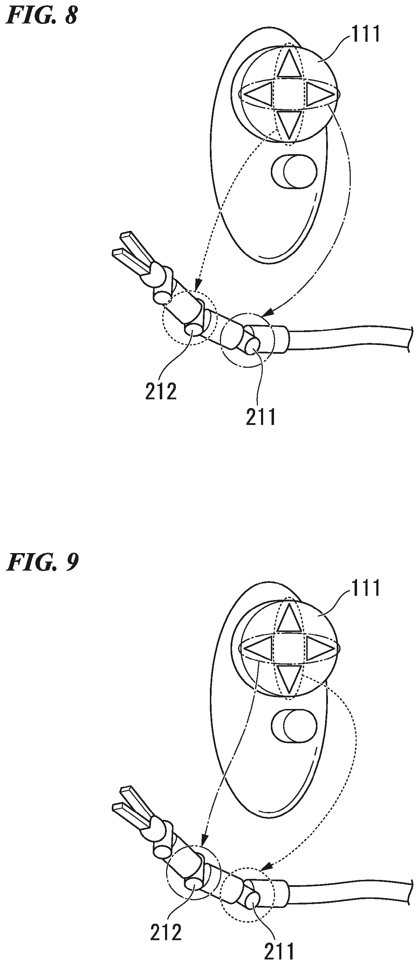

As an example, in first correspondence information shown in Fig. 8, the input in the right and left directions of the direction switch 111 and the joint 211 are associated with each other, and the input in the up and down directions and the joint 212 are associated with each other. Meanwhile, in second correspondence information shown in Fig. 9, the input in the up and down directions of the direction switch 111 and the joint 211 are associated with each other, and the input in the left and right directions and the joint 212 are associated with each other, and accordingly the association is inverted from the first correspondence information. Thus, when the plurality of pieces of correspondence information are prepared in a single treatment tool, the medical manipulator may be configured such that the list of the correspondence information and the content of each piece of correspondence information are displayed on the display 29 or the like to be selected by the operator.

Furthermore, in the plurality of provided treatment tools, the number, the degree of freedom, or the like of the joints as well as the end effecter may be different from each other.

Further, the manipulation input unit may wirelessly send the manipulation input to the master processing circuit. In this case, the correspondence information may be wirelessly sent to the controller.

Further, in the present invention, when the treatment tool and the manipulation input unit are disposable, for example, the storage unit is configured to be able to record and update the number of connections with the controller. Further, the control unit may be configured such that, when the treatment tool is connected, the number of connections recorded on the storage unit is obtained, and when the number of connections is equal to or greater than a predetermined value, use of the connected treatment tool is not allowed, and a message, a warning sound, or the like is reported to the operator.

The present invention is not limited by the foregoing description, but is only limited by the appended claims.

### Industrial Applicability

According to the present invention, it is possible to provide a medical manipulator that is capable of reliably providing the operating parameters or the like corresponding to a treatment tool to the control unit, without being affected by the backflow of blood, a body fluid, or the like. Further, it is possible to provide a treatment tool package that can be suitably applied to the medical manipulator.

### Reference Signs List

- 1:: Medical manipulator
- 2, 2A, 2B, 2C:: Treatment tool
- 10A, 10B:: Motor unit (driving unit)
- 11:: Manipulation input unit
- 11 A, 11B, 11C:: Master unit (manipulation input unit)
- 15:: Controller
- 50, 50A:: Packaging material
- 70, 70A, 80, 90:: Treatment tool package
- 115:: Storage unit
- 116:: SD memory card (storage unit, storage medium)
- 117:: Barcode (storage unit)

## Claims

1. A medical manipulator equipped with a manipulation input unit by which an operator performs a manipulation input, and a treatment tool that is driven in accordance with the manipulation input, the medical manipulator comprising:
a storage unit having correspondence information used when a driving signal that drives the treatment tool is generated in accordance with the manipulation input,
wherein the storage unit is disposed at the manipulation input unit.

2. The medical manipulator according to claim 1, wherein the storage unit is attachable to and detachable from the manipulation input unit.

3. The medical manipulator according to claim 1, wherein the storage unit is a barcode that is printed on the manipulation input unit.

4. The medical manipulator according to any one of claims 1 to 3, wherein the treatment tool and the manipulation input unit are disposable elements that are discarded after use.

5. The medical manipulator according to any one of claims 1 to 4, wherein the storage unit has a plurality of pieces of the correspondence information.

6. A medical manipulator equipped with a manipulation input unit by which an operator performs a manipulation input, and a treatment tool that is driven in accordance with the manipulation input, the medical manipulator comprising:
a controller to which the treatment tool is connected; and
a storage unit having correspondence information used when a driving signal that drives the treatment tool is generated in accordance with the manipulation input,
wherein the storage unit is attachable to and detachable from the controller.

7. A medical manipulator equipped with a manipulation input unit by which an operator performs a manipulation input, and a treatment tool that is driven in accordance with the manipulation input, the medical manipulator comprising:
a driving unit to which the treatment tool is connected; and
a storage unit having correspondence information used when a driving signal that drives the treatment tool is generated in accordance with the manipulation input,
wherein the storage unit is attachable to and detachable from the driving unit.

8. A treatment tool packages used in a medical manipulator, comprising:
a manipulation input unit by which an operator performs a manipulation input;
a treatment tool that is driven in accordance with the manipulation input;
a storage unit having correspondence information used when a driving signal that drives the treatment tool is generated in accordance with the manipulation input; and
a packaging material that houses the treatment tool and the manipulation input unit.

9. The treatment tool package according to claim 8, wherein the storage unit is provided at the manipulation input unit.

10. The treatment tool package according to claim 8, wherein the storage unit is a storage medium that is housed in the packaging material.

11. The treatment tool package according to claim 8, wherein the storage unit is a barcode that is printed on the packaging material.
